# EUROPEAN PATENT APPLICATION

(11) **EP 4 029 931 A1**
(43) Date of publication of application: **20.07.2022**
(21) Application number: 21217104.5
(22) Date of filing: 22.12.2021
(51) Int. Cl.: C12N 1/20, C12P 19/12

(54) **ALGINATE LYASE-PRODUCING GENETIC ENGINEERED STRAIN AND FERMENTATION METHOD THEREOF**

(30) Priority: 25.12.2020 CN 202011558467
(71) Applicant: Beijing Leili Marine Bioindustry Inc., Beijing (CN)
(72) Inventor: Tang, Jie, Haidian District, Beijing (CN); Yan, Guofu, Haidian District, Beijing (CN)
(74) Representative: Geskes, Christoph

(57) **Abstract**

The invention relates to the technical field of microorganisms, in particular to an alginate lyase-producing genetic engineered strain and a fermentation method thereof. The sequence of the 16S rDNA transferred into the alginate lyase-producing genetic engineered strain is shown as SEQ ID NO:1. The strain of the invention has the advantages of fast growth, high enzyme activity, good enzyme stability, strong adaptability to substrates from different sources, etc., and therefore has good application prospects.

SEQUENCE LISTING

<110>  Beijing Leili Marine Bioindustry Inc.

<120>  ALGINATE LYASE-PRODUCING GENETIC ENGINEERED STRAIN AND FERMENTATION
METHOD THEREOF

<130>  0653PA21002EP

<150>  CN202011558467X
<151>  2020-12-24

<160>  1

<170>  SIPOSequenceListing 1 .0

<210>  1
<211>  1448
<212>  DNA
<213>  Artificial Sequence

<400>  1
ggcgcaggcc tacacatgca agtcgagcgg aacgagttat ctgaaccttc gggggacgat     60

aacggcgtcg agcggcggac gggtgagtaa tgcctaggaa attgccctga tgtgggggat     120

aaccattgga aacgatggct aataccgcat gatgcctacg ggccaaagag ggggaccttc     180

gggcctctcg cgtcaggata tgcctaggtg ggattagcta gttggtgagg taagggctca     240

ccaaggcgac gatccctagc tggtctgaga ggatgatcag ccacactgga actgagacac     300

ggtccagact cctacgggag gcagcagtgg ggaatattgc acaatgggcg caagcctgat     360

gcagccatgc cgcgtgtgtg aagaaggcct tcgggttgta aagcactttc agtcgtgagg     420

aaggtggtgt agttaatagc tgcattattt gacgttagcg acagaagaag caccggctaa     480

ctccgtgcca gcagccgcgg taatacggag ggtgcgagcg ttaatcggaa ttactgggcg     540

taaagcgcat gcaggtggtt tgttaagtca gatgtgaaag cccggggctc aacctcggaa     600

tagcatttga aactggcaga ctagagtact gtagaggggg gtagaatttc aggtgtagcg     660

gtgaaatgcg tagagatctg aaggaatacc ggtggcgaag gcggccccct ggacagatac     720

tgacactcag atgcgaaagc gtggggagca aacaggatta gataccctgg tagtccacgc     780

cgtaaacgat gtctacttgg aggttgtggc cttgagccgt ggctttcgga gctaacgcgt     840

taagtagacc gcctggggag tacggtcgca agattaaaac tcaaatgaat tgacgggggc     900

ccgcacaagc ggtggagcat gtggtttaat tcgatgcaac gcgaagaacc ttacctactc     960

ttgacatcca gagaactttc cagagatgga ttggtgcctt cgggaactct gagacaggtg     1020

ctgcatggct gtcgtcagct cgtgttgtga aatgttgggt taagtcccgc aacgagcgca     1080

acccttatcc ttgtttgcca gcgagtaatg tcgggaactc cagggagact gccggtgata     1140

aaccggagga aggtggggac gacgtcaagt catcatggcc cttacgagta gggctacaca     1200

cgtgctacaa tggcgcatac agagggcggc caacttgcga aagtgagcga atcccaaaaa     1260

gtgcgtcgta gtccggattg gagtctgcaa ctcgactcca tgaagtcgga atcgctagta 1320

atcgtggatc agaatgccac ggtgaatacg ttcccgggcc ttgtacacac cgcccgtcac 1380

accatgggag tgggctgcaa aagaagtagg tagtttaacc ttcgggggga cgctaccact 1440

tggttccg 1448

## Description

### FIELD OF THE APPLICATION

The invention relates to the technical field of microorganisms, in particular to an alginate lyase-producing genetic engineered strain and a fermentation method thereof.

### BACKGROUND

Alginate is an acidic marine polysaccharide with abundant sources and wide-ranging applications. It is linear high molecular weight polymer formed by a random combination of α-L-1,4 guluronic acid (G) and β-D-1,4 mannuronic acid (M). With the discovery of multiple physiological activities of Alginate oligosaccharides, the huge application potential of Alginate oligosaccharides has attracted more and more attention. In recent years, the research on the biological activity of Alginate oligosaccharides has made significant progress, such as regulating plant growth, inducing plant disease resistance, anti-tumor and anti-senile dementia, and preventing and treating common diseases of marine invertebrates. Therefore, alginate oligosaccharides prepared by various degradation methods have important research value in the fields of sugar chemistry, glycobiology, glycoengineering and carbohydrate drug research.

At present, the methods for obtaining Alginate oligosaccharides mainly include physical degradation, acid degradation, oxidative degradation and enzymatic hydrolysis. Among them, enzymatic hydrolysis is a biodegradation method with mild conditions, strong controllability and high specificity, and it also represents a main research direction. Alginate lyase degrades alginate through β elimination mechanism. It is not only used as a tool enzyme for the preparation of Alginate oligosaccharides, but also has certain medicinal value. Alginate lyase can degrade extracellular alginate polysaccharide biomembrane to restore the sensitivity of pathogenic bacteria to antibiotics, which is effective in the treatment of lung infections in patients with cystic fibrosis (CF). In addition, Alginate lyase is also used in the preparation of algae protoplasts, which plays an important role in basic research on the physiological and biochemical characteristics of algae.

Alginate lyases have a wide range of sources, and more than 50 alginate lyases have been found, which are mainly from marine algae, molluscs, marine bacteria, terrestrial fungi, and a few phages and viruses. The Alginate lyase derived from microorganisms is mainly derived from marine bacteria and fungi. Enzyme-producing strains can be obtained through enrichment of brown algae epiphytic bacteria and screening, but most of the strains have low enzyme production levels. For example, the alginate lyase produced by Acinetobacter X8 is 157.3 U/mL and 114.8 U/mL, and 6.48U/mL (UV method) , respectively; and these enzymes are affected by the specificity of the substrates and have fewer sites for the degradation of Alginate, which is the main limiting factor for the enzymatic preparation of Alginate oligosaccharides. So no industrialization has been achieved for any enzyme. Therefore, screening new strains for high-efficiency degrading alginate and looking for new high-activity Alginate lyase have important theoretical significance and well application prospects.

### SUMMARY OF THE INVENTION

In view of this, the present invention provides an alginate lyase-producing genetic engineered strain and a fermentation method thereof. The strain has the advantages such as fast growth, high enzyme activity, good enzyme stability, and strong adaptability to substrates from different sources.

In order to achieve the above-mentioned purpose of the invention, the present invention provides the following technical solutions:

According to one aspect, there is provided 16S rDNA, and its sequence is shown as SEQ ID NO:1.

According to another aspect, there is provided application of 16S rDNA in the preparation of alginate lyase-producing genetic engineered strain.

According to yet another aspect, there is provided a plasmid, which has the above-mentioned 16S rDNA.

In one embodiment, the vector of the plasmid is a PSKH plasmid.

According to yet another aspect, there is provided a genetically engineered strain, which comprises the above-mentioned 16S rDNA, or the above-mentioned plasmid.

According to yet another aspect, there is provided a method for culturing the genetically engineered strain by using agar culture medium or liquid culture medium to cultivate the genetically engineered strain.

In one embodiment, the composition of agar culture medium is: nutrient agar 2 to 5 g, sodium chloride 0.5 to 1 g, dipotassium hydrogen phosphate 0.3 to 1 g, sodium alginate 0.5 to 1 g, and water 100 mL.

In one embodiment, the conditions for culturing by using agar culture medium are: 28-32 °C for at least 10 hours.

In one embodiment, the composition of liquid culture medium is: peptone 1-3g, ammonium sulfate 0.2-0.5g, sodium chloride 1-3g, dipotassium hydrogen phosphate 0.3-1g, magnesium sulfate 0.02-0.05g, Tween 80 0.05~0.2mL, water 100mL.

In one embodiment, the conditions of culture with the liquid culture medium are: 28~32 °C, rotation speed of160~200r·min⁻¹ for at least 10h.

According to yet another aspect, there is provided a fermentation method of the genetically engineered strain, comprising using a fermentation culture medium to cultivate the genetically engineered strain.

In one embodiment, the composition of the fermentation culture medium is: sodium alginate 0.6-1.5%, Spray Drying Corn Steep Liquor 0.8-1.2%, ammonium sulfate 0.2-0.5%, Sodium chloride 1 to 3%, dipotassium hydrogen phosphate is 0.3 to 1%, magnesium sulfate 0.02 to 0.05%, and the pH is 7.0 to 7.5.

In one embodiment, the conditions for culture with the fermentation medium are: 28-32 °C, rotation speed of160-200 r·min⁻¹ for at least 10 hours.

In one embodiment, the fermentation production method of alginate lyase includes the following steps:
(1) Preparing seed liquid;
(2) Inoculating the seed liquid prepared in step (1) into 300-500L fermentation culture medium for propagation;
(3) The culture obtained in step (2) is inoculated to the fermentation culture medium for fermentation culture.

The present invention provides an alginate lyase-producing genetic engineered strain and a fermentation method thereof. The sequence of 16S rDNA transferred into the alginate lyase-producing genetic engineered strain is shown as SEQ ID NO:1. The technical effects of the present invention are:

The strain of the present invention has the advantages of fast growth, high enzyme activity, good enzyme stability, and strong adaptability to substrates from different sources, and therefore has good application prospects.

The present invention uses the fusion PCR technology to repeatedly clone 16S DNA fragment, and the enzyme activity doubled, which proves that the gene fragment is an active fragment and plays a key role.

### BRIEF DESCRIPTION OF THE DRAWINGS

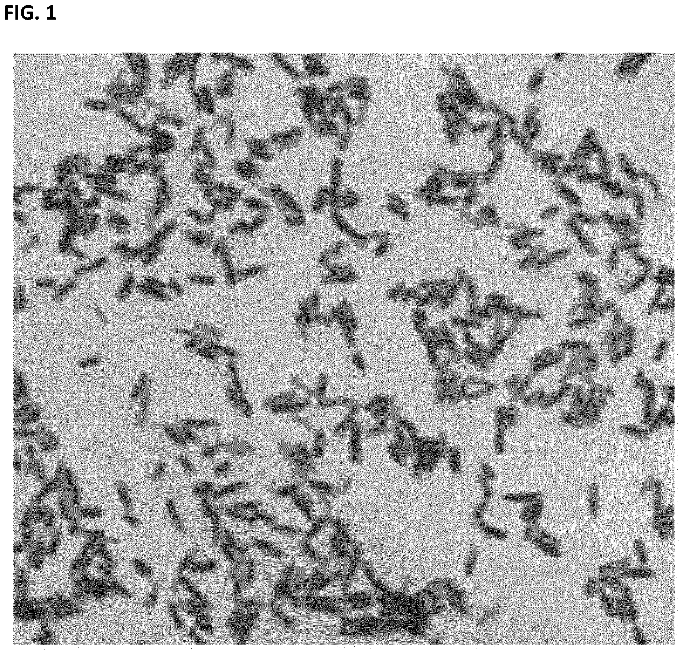

Figure 1 is 1000 times micrograph of the alginate lyase-producing strain.

### DETAILED DESCRIPTION OF THE INVENTION

The present invention discloses an alginate lyase-producing genetic engineered strain and a fermentation method thereof. Those skilled in the art can learn from the content of this application and appropriately improve the process parameters. In particular, it should be pointed out that all similar substitutions and modifications are obvious to those skilled in the art, and they are all considered to be included in the present invention. The method and application of the present invention have been described through the preferred embodiments. It is obvious that one skilled in the art can make modifications or appropriate changes and combinations to the methods and applications described herein without departing from the content, spirit and scope of the present invention, which will fall within the scope of the present invention.

The materials and reagents involved in the following examples for preparing the alginate lyase-producing genetic engineered strain and carrying out the fermentation method are commercially available.

The present invention will be further explained below in conjunction with examples:

### Example 1. Screening method and identification of strains

### (1) Domestication and enrichment of strains

10 grams of rotten Sargassum was taken and put into 100 ml of sterile normal saline to prepare a bacterial suspension, and 10 mL of the bacterial suspension was transferred into 200 mL of a domestication and enrichment medium containing 10 g/L of sodium alginate, and cultured for 24 hours to obtain a domestication and enrichment solution; 10 mL of the domestication and enrichment solution was taken to fresh domestication and enrichment medium to make a total of 8 generations of domestication. In the last three generations of domestication, the amount of sodium alginate added was further increased to 15g/L~20g/L.

### (2) initial Screening

The domestication liquid obtained in step (1) was diluted with sterile seawater according to different concentration gradient of 10⁻¹ to 10⁻⁶, and0.1 mL of each diluents was took and applied to the primary screening separation plate, and was cultured at a constant temperature of 25 °C for 72 hours, and then colonies with transparent circles were selected for separation and purification by streaking, and transferred to a slant for preservation.

### (3) further screening

The initially screened colonies were inoculated to 100mL seed culture medium (250mL Erlenmeyer flask), and cultured at 30 °C and rotation speed of 180r·min-1 for 22h; 5mL of seed liquid cultured to the logarithmic phase was taken and inoculated to 200mL fermentation medium (500mL Erlenmeyer flask) for fermentation culture and cultured at 30 °C and rotation speed of 180r·min-1 for 22h, and then centrifuged at 8000r·min-1 and 4 °C for 10min; the supernatant was taken to measure the enzyme activity by DNS method.

Liquid culture medium composition: peptone 1g, ammonium sulfate 0.3g, sodium chloride 2g, dipotassium hydrogen phosphate 1g, magnesium sulfate 0.05g, Tween 80 0.1mL, tap water 100mL.

Fermentation medium composition: sodium alginate 0.8%, Spray Drying Corn Steep Liquor 1%, ammonium sulfate 0.3%, sodium chloride 2%, dipotassium hydrogen phosphate 1%, magnesium sulfate 0.05%, pH is 7. 0.

The morphological, physiological and biochemical characteristics of the strain of the present invention are as follows:
Colony color: light yellow.
Aerobic mode: aerobic growth.
Colony size: 5-8mm.
Suitable growth temperature: 28-32 °C.
Suitable growth pH: 6.8 to 7.3.
Morphology: round colony, moist and luster.
Gram stain: positive.

### (4) Determination of the activity of alginate lyase

An improved DNS (3,5-dinitrosalicylic acid) method was used. Due to the thermal instability of sodium alginate, the DNS method was appropriately improved.0.1mL enzyme solution was taken and added to 1mL 1% sodium alginate solution (prepared with Na2HPO4-NaH2PO4 buffer solution, 0.05mol/L, pH 7.0); After reaction at 40 °C for 20min, 1mL DNS solution was added; After reaction in boiling water bath for 3min; the solution was cooled quickly, the volume thereof was adjusted to 10 mL, and the absorbance was measured at 540 nm with a visible light spectrophotometer. A standard curve was drawn with Anhydrous glucose, and the amount of reducing sugar produced was calculated based on the difference between the reaction group and the control group. One active unit of enzyme activity is defined as the amount of enzyme required to produce 1 µmol reducing sugar per minute.

### (5) Identification of enzyme

Fermentation broth for producing enzyme by the strain was identified. The suitable temperature of the enzyme was 35-40 °C, and the suitable pH was 7.0-8.0. The optimal temperature of the enzyme was determined to be 40 °C, and the optimal pH was 7.5. The strain secretes extracellular enzymes, which have a good degrading effect on soluble macromolecular alginates from different sources.

### (6) Microscopic characteristics of strains and 16S rDNA sequencing

The micrographs of the strains are shown in Figure 1.

The inactivated bacterial solution was prepared and submitted to Beijing Huada Gene Technology Co., Ltd (BGI) for 16S rDNA sequencing. The 16S rDNA (ITS-5.8S-ITS2 region) sequence of this strain is:

GGCGCAGGCCTACACATGCAAGTCGAGCGGAACGAGTTATCTGAACCTTCGGGGGACGATAACGGCGT
CGAGCGGCGGACGGGTGAGTAATGCCTAGGAAATTGCCCTGATGTGGGGGATAACCATTGGAAACGATGGCTAATA
CCGCATGATGCCTACGGGCCAAAGAGGGGGACCTTCGGGCCTCTCGCGTCAGGATATGCCTAGGTGGGATTAGCTA
GTTGGTGAGGTAAGGGCTCACCAAGGCGACGATCCCTAGCTGGTCTGAGAGGATGATCAGCCACACTGGAACTGAG
ACACGGTCCAGACTCCTACGGGAGGCAGCAGTGGGGAATATTGCACAATGGGCGCAAGCCTGATGCAGCCATGCCG
CGTGTGTGAAGAAGGCCTTCGGGTTGTAAAGCACTTTCAGTCGTGAGGAAGGTGGTGTAGTTAATAGCTGCATTAT
TTGACGTTAGCGACAGAAGAAGCACCGGCTAACTCCGTGCCAGCAGCCGCGGTAATACGGAGGGTGCGAGCGTTAA
TCGGAATTACTGGGCGTAAAGCGCATGCAGGTGGTTTGTTAAGTCAGATGTGAAAGCCCGGGGCTCAACCTCGGAA
TAGCATTTGAAACTGGCAGACTAGAGTACTGTAGAGGGGGGTAGAATTTCAGGTGTAGCGGTGAAATGCGTAGAGA
TCTGAAGGAATACCGGTGGCGAAGGCGGCCCCCTGGACAGATACTGACACTCAGATGCGAAAGCGTGGGGAGCAAA
CAGGATTAGATACCCTGGTAGTCCACGCCGTAAACGATGTCTACTTGGAGGTTGTGGCCTTGAGCCGTGGCTTTCG
GAGCTAACGCGTTAAGTAGACCGCCTGGGGAGTACGGTCGCAAGATTAAAACTCAAATGAATTGACGGGGGCCCGC
ACAAGCGGTGGAGCATGTGGTTTAATTCGATGCAACGCGAAGAACCTTACCTACTCTTGACATCCAGAGAACTTTC
CAGAGATGGATTGGTGCCTTCGGGAACTCTGAGACAGGTGCTGCATGGCTGTCGTCAGCTCGTGTTGTGAAATGTT
GGGTTAAGTCCCGCAACGAGCGCAACCCTTATCCTTGTTTGCCAGCGAGTAATGTCGGGAACTCCAGGGAGACTGC
CGGTGATAAACCGGAGGAAGGTGGGGACGACGTCAAGTCATCATGGCCCTTACGAGTAGGGCTACACACGTGCTAC
AATGGCGCATACAGAGGGCGGCCAACTTGCGAAAGTGAGCGAATCCCAAAAAGTGCGTCGTAGTCCGGATTGGAGT
CTGCAACTCGACTCCATGAAGTCGGAATCGCTAGTAATCGTGGATCAGAATGCCACGGTGAATACGTTCCCGGGCC
TTGTACACACCGCCCGTCACACCATGGGAGTGGGCTGCAAAAGAAGTAGGTAGTTTAACCTTCGGGGGGACGCTAC
CACTTGGTTCCG。

And according to the blast results, this strain has the closest Genetic relationship with Vibrio alginolyticus.
Source Vibrio alginolyticus
Source: Vibrio alginolyticus
   Bacteria; Proteobacteria; Gammaproteobacteria; Vibrionales;
   Vibrionaceae; Vibrio.

### Results: The activity of the alginate lyase is 1800 U/mL.

### Example 2:

The upstream and downstream homologous arms of the 16S rDNA fragment in Example 1 of the target gene were fused and cloned into the PSKH plasmid by fusion PCR technology, the recombinant plasmid was introduced into E. coli S17λpir, and then delivered into Vibrio parahaemolyticus strain by conjugation, the mutant strain was obtained by reverse screening of the sacB gene on pDS132 plasmid.

Culture and fermentation for the mutant strain: the culturing method or fermentation method used for the mutant strain is the same as in Example 1.

Results: The activity of the alginate lyase is 3610 U/mL. Compared with Example 1, the process conditions are completely the same, while the 16S DNA fragment of the target gene is repeatedly cloned by fusion PCR technology, and the enzyme activity is doubled, so that it can be determined that the gene fragment is an active fragment, which plays a key role.

### Example 3. Methods for strain activation, preservation, and preparation of fermentation strains

### (1) Strain activation

An improved nutrient agar medium was used, and the composition was: nutrient agar 2g, sodium chloride 0.5g, dipotassium hydrogen phosphate 0.8g, sodium alginate 0.5g, and tap water 100mL. A solid slant culture medium was prepared , a small amount of strain was picked with an inoculating needle and streaked on the above-mentioned solid slant medium, and then placed in a constant temperature incubator at 30 °C for 24 hours.

### (2) Strain preservation

An improved nutrient agar medium was used, and the composition was: nutrient agar 2g, sodium chloride 0.5g, dipotassium hydrogen phosphate 0.8g, sodium alginate 0.5g, and tap water 100mL. Prepared a solid slant culture medium, an inoculating needle was used to pick or dip a small amount of strains bacteria to be preserved on the solid slant culture medium, and placed in a constant temperature incubator at 30 °C for 24 hours, and then stored in a refrigerator at 4 °C . It can be effectively preserved for 1 month, and long-term preservation needs a freeze-dried tube form for liquid nitrogen preservation.

### (3) Liquid propagation

a test tube with activated strains was taken, washed with sterile physiological saline, transferred all to 100-1000 ml of liquid propagation medium, and incubated at 30 °C and 180 rad/min for 24 hours. The composition of Liquid propagation medium: peptone 1g, ammonium sulfate 0.3g, sodium chloride 2g, dipotassium hydrogen phosphate 1g, magnesium sulfate 0.05g, Tween 800.1mL, tap water 100mL.

### Example 4. Method for producing algin lyase by fermentation of strains

### (1) Strain activation

An improved nutrient agar medium was used, and the composition was: nutrient agar 2 g, sodium chloride 0.5 g, dipotassium hydrogen phosphate 0.8 g, sodium alginate 0.5 g, and tap water 100 mL. A solid slant medium was prepared, an inoculating needle was used to pick or dip a small amount of preserved strains on the solid slant medium, and then placed it in a constant temperature incubator at 30 °C for 24 hours.

### (2) Liquid expansion

a test tube with activated strains was taken, wash them with sterile physiological saline, and transfer all to 100-1000 ml of liquid expansion medium, and cultured at 30 °C and 180 rad/min for 24 hours. The composition of Liquid medium formula: peptone 1g, ammonium sulfate 0.3g, sodium chloride 2g, dipotassium hydrogen phosphate 1g, magnesium sulfate 0.05g, Tween 800.1mL, tap water 100mL.

(3) the seed liquid prepared in step (2) was inoculated into 300L of liquid fermentation medium for propagation; the cultivation temperature is 30 °C, the pressure is 0.1 MPa, the stirring frequency is 50 Hz/min, and the time of cultivation is 18-24 hours.

(4) The culture cultured in step (3) is inoculated to a large amount of fermentation medium for fermentation culture.

Wherein, the composition of fermentation medium in step (4): sodium alginate 0.8%, Spray Drying Corn Steep Liquor 1%, ammonium sulfate 0.3%, sodium chloride 2%, dipotassium hydrogen phosphate 1%, sulfuric acid Magnesium 0.05%, pH 7.0. The fermentation medium in step (4) is the same as the medium in step (3).

The above are only the preferred embodiments of the present invention. It should be pointed out that for those of ordinary skill in the art, without departing from the principle of the present invention, improvements and modifications can be made. These improvements and modifications should also be regarded as falling in the protection scope of the present invention.

## Claims

1. 16S rDNA, wherein its sequence is shown as SEQ ID NO:1.

2. Use of 16S rDNA of claim 1 in the preparation of alginate lyase-producing genetic engineered strain.

3. A plasmid, wherein it comprises 16S rDNA of claim 1.

4. The plasmid according to claim 3, wherein the vector of the plasmid is a PSKH plasmid.

5. A genetic engineered strain, wherein it comprises 16S rDNA of claim 1 or the plasmid of claim 3 or 4.

6. A method for culturing the genetic engineered strain of claim 5, wherein the genetic engineered strain is cultured using an agar culture medium or a liquid culture medium.

7. The method according to claim 6, wherein
- the composition of the agar culture medium is: nutrient agar 2-5g, sodium chloride 0.5-1g, dipotassium hydrogen phosphate 0.3-1g, sodium alginate 0.5~1g, and water 100mL;
- the conditions for culture with agar medium are: 28~32°C for at least 10h.

8. The method according to claim 6, wherein
- the composition of the liquid culture medium is: peptone 1 to 3 g, ammonium sulfate 0.2 to 0.5 g, sodium chloride 1 to 3 g, dipotassium hydrogen phosphate 0.3~1g, magnesium sulfate 0.02~0.05g, Tween 80 0.05~0.2mL, and water 100mL;
- the conditions of culture with the liquid culture medium are: 28~32°C, rotation speed of160~200r·min⁻¹ for at least 10h.

9. A method for fermentation of the genetic engineered strain of claim 5, wherein the genetic engineered strain is cultured by using a fermentation medium.

10. The method according to claim 9, wherein the composition of the fermentation medium is: sodium alginate 0.6-1.5%, Spray Drying Corn Steep Liquor 0.8-1.2%, ammonium sulfate 0.2 to 0.5%, sodium chloride 1 to 3%, dipotassium hydrogen phosphate 0.3 to 1%, magnesium sulfate 0.02 to 0.05% ; pH is 7.0 to 7.5; the conditions of fermentation culture are: 28~32°C, rotation speed of160~200r·min⁻¹ for at least 10h.
